# EUROPEAN PATENT APPLICATION

(11) **EP 4 725 471 A1**
(43) Date of publication of application: **15.04.2026**
(21) Application number: 25203977.1
(22) Date of filing: 23.09.2025
(51) Int. Cl.: A61K 8/04, C08B 31/04, C08B 37/00, C08L 3/02

(54) **METHOD OF PREPARING DEXTRIN FATTY ACID ESTER, TRANSPARENT OIL GEL CONTAINING THE DEXTRIN FATTY ACID ESTER PRODUCED THEREFROM, AND TRANSPARENT SOLID COSMETIC MATERIAL USING THE SAME**

(30) Priority: 14.10.2024 KR 20240139388
(71) Applicant: Shinsung Materials Co., Ltd., Chungcheongbuk-do 27850 (KR)
(72) Inventor: KIM, Kwang Shik, 27850 Jincheon-gun, Chungcheongbuk-do (KR); SHIN, Sang Kyu, 27850 Jincheon-gun, Chungcheongbuk-do (KR)
(74) Representative: BCKIP Part mbB

(57) **Abstract**

The present invention relates to a method for preparing a dextrin fatty acid ester that involves dispersing dextrin in an organic mixed solvent containing a hexane-based solvent, adding a fatty acid halide for reaction, and after the reaction, adding water to induce layer separation to isolate the supernatant, and precipitating crystals from the supernatant to provide a dextrin fatty acid ester, in particular, by providing a transparent dextrin palmitate with high gel strength, a transparent oil gel formulated with said dextrin palmitate and oil, and various transparent solid cosmetic materials using it can be provided.

## Description

### BACKGROUND OF THE INVENTION

### FIELD OF THE INVENTION

The present invention relates to a method of preparing a dextrin fatty acid ester, a transparent oil gel containing the dextrin fatty acid ester preparing therefrom, and a transparent solid cosmetic material using the same, and more particularly, to a method of preparing a dextrin fatty acid ester, comprising: dispersing dextrin in an organic mixed solvent containing a hexane-based solvent, adding a fatty acid halide to carry out a reaction, and after the reaction is completed, adding water to induce layer separation to separate an supernatant, and precipitating crystals from the supernatant to provide a dextrin fatty acid ester, particularly a transparent dextrin palmitate having a strong gel strength, a transparent oil gel containing the dextrin palmitate and an oil, and a transparent solid cosmetic material using the same.

### DESCRIPTION OF THE RELATED ART

Oils are used as ingredients in transparent cosmetics, offering advantages such as an aesthetic appearance and a transparent finish upon application. In particular, dextrin fatty acid esters have been used as a representative cosmetic ingredient for forming transparent oil gels.

For example, Patent Document 1 discloses a transparent cosmetic material implemented by combining a dextrin fatty acid ester, a heavy liquid isoparaffin, and a liquid oil to improve gloss upon application, and the dextrin fatty acid ester is characterized by the use of dextrin palmitate.

Patent Document 2 discloses a widely known transparent cosmetic formulation containing 12-hydroxystearic acid or dextrin fatty acid ester. However, these formulations exhibit a dripping texture upon application and lack sufficient finish after application, resulting in a decrease in transparency over time. Furthermore, the transparent cosmetic formulation containing a dextrin fatty acid ester lacks sufficient stick formability, and the addition of a solidifying agent such as wax to overcome this also results in a decrease in transparency.

To address these issues, Patent Document 2 discloses a gel composition including a dextrin fatty acid ester in a transparent cosmetic formulation containing 12-hydroxystearic acid and an oil. The gel composition includes one or more dextrin fatty acid esters selected from the group consisting of dextrin palmitate, dextrin 2-ethylhexanoate, and dextrin myristate/palmitate.

Patent Document 3 discloses oil-based solid cosmetic materials such as foundations, make-up base foundations, lipsticks, eye shadows, and lip creams. These solid cosmetic materials require excellent transparency, a non-sticky feeling upon application, and a refreshing feeling after application as well as good extensibility and storage stability.

To meet these requirements, Patent Document 3 discloses a transparent solid cosmetic including a dextrin fatty acid ester (dextrin palmitate), a liquid oil with a hydroxyl value of 20 or less, amorphous fine particles of anhydrous silicic acid with a particle diameter of 0.001 to 0.05 µm, and spherical anhydrous silicic acid with a particle size of 0.1 to 30 µm.

Patent document 4 relates to a cleansing cosmetic including a dextrin fatty acid ester, a surfactant, and an oil ingredient. The dextrin fatty acid ester is included to maintain a transparent gel phase without any risk of flowing, and the structure gradually melts due to the body temperature and friction upon skin application, thereby providing a feeling of use similar to that of a conventional cleansing oil. Therefore, a transparent/translucent cleansing cosmetic composition that is easy to use is provided, since it does not flow upon skin application.

Another approach to providing a transparent solid cosmetic is disclosed in Patent Document 5, which proposes a transparent solid cosmetic prepared by using an amino acid-based gelling agent such as dibutyl lauroyl glutamide (GP-1) and dibutyl hexanoyl glutamide (EB-21) and increasing the temperature to 120 to 140 °C when forming an oil and gel to form an oil gel. However, when using a gelling agent as described above, the gel is formed at a high temperature, which causes the cosmetic composition ingredients to be oxidized or decomposed, making it difficult to prepare the desired cosmetic.

Furthermore, Patent Document 6 relates to a low-friction oil-based cleanser composition, which is a makeup cleansing agent, containing an ester-based oil and a dextrin-based oil thickener as active ingredients, and the dextrin-based oil thickener is dextrin palmitate/ethylhexanoate included in an amount of 0.5% to 3.0 % by weight based on the total weight of the composition.

Therefore, among dextrin fatty acid esters, dextrin palmitate/ethylhexanoate and dextrin myristate form an oil gel that is transparent to some extent. However, there are problems when they are used alone as an oil gelling agent in various oils such as mineral oils, and therefore, they are often mixed with dextrin palmitate.

Conventionally, dextrin fatty acid esters have been used to prepare transparent cosmetics, and dextrin palmitate, in particular, is currently available on the market. However, while a gel strength of approximately 200 g is maintained in mineral oils, the transparency is significantly reduced.

Therefore, the present inventors have endeavored to address the problem inherent in dextrin fatty acid esters, particularly dextrin palmitate, that the oil gel prepared therefrom is not transparent and have obtained a transparent gel by mixing dextrin palmitate with an oil and confirmed that a transparent oil gel was implemented by mixing dextrin palmitate alone with an oil and that a high gel strength was achieved, thereby completing the present invention.

[Related Art Documents]
(Patent Document 1) Japanese Patent Publication No. 1997-235210 (published on September 9, 1997, Title: Solid cosmetic material)
(Patent Document 2) Japanese Patent Publication No. 2001-39817 (published on February 13, 2001, Title: Transparent cosmetic)
(Patent Document 3) Japanese Patent Publication No. 1999-255616 (published on September 21, 1999, Title: Solid cosmetic material)
(Patent Document 4) Korean Patent Publication No. 2015-0014279 (published on February 6, 2015, Title: Transparent or translucent gel-shaped cleansing cosmetic composition)
(Patent Document 5) Korean Patent Publication No. 2610444 (published on December 1, 2023, Title: Cosmetic composition)
(Patent Document 6) Korean Patent Publication No. 1877252 (published on July 11, 2018, Title: Low-friction oil cleanser composition)

### SUMMARY OF THE INVENTION

An object of the present invention is to provide a method of preparing a dextrin fatty acid ester.

Another object of the present invention is to provide a transparent oil gel formulated with the dextrin fatty acid ester prepared by the above method and an oil.

A further object of the present invention is to provide a transparent solid cosmetic material containing a solid composition using the said transparent oil gel as a base.
To achieve the above-mentioned objects, the present invention provides a method of preparing a dextrin fatty acid ester, comprising: a first step of dispersing dextrin in an organic mixed solvent containing a hexane-based solvent, and then adding a fatty acid halide while maintaining a temperature of 0 to 120°C to carry out a reaction;
a second step of adding water after the completion of the reaction to induce layer separation and separating the supernatant; and
a third step of precipitating and drying crystals from the supernatant,
wherein the organic mixed solvent has different polarities, and in the second step, the hexane-based solvent remains in the supernatant upon phase separation by water, thereby separating the dextrin fatty acid ester.

The organic mixed solvent includes a first solvent selected from the group consisting of n-hexane, cyclohexane, and methylcyclohexane and a second solvent selected from the group consisting of dimethylformamide, dimethylacetamide, dimethyl sulfoxide (DMSO), and N-methyl-2-pyrrolidone (NMP), and preferably, an embodiment of the present invention is implemented under the conditions of an organic mixed solvent including methylcyclohexane and dimethylformamide.

Preferably, the dextrin in the first step has an average saccharide degree of polymerization of 35 to 80.

In addition, in the dextrin fatty acid ester of the present invention, the fatty acid is a higher fatty acid having 12 to 22 carbon atoms, and more preferably, dextrin palmitate is prepared in an embodiment of the present invention. In this case, the dextrin palmitate has an average degree of acylation substitution of 1.3 to 2.5.

The present invention provides a transparent oil gel comprising 2 to 20% by weight of a dextrin fatty acid ester prepared by the method of any one of claims 1 to 7, and 80 to 98% by weight of oil.

More preferably, the dextrin fatty acid ester is dextrin palmitate, and the gel has a transmittance of 10% or more at a wavelength of 375 nm when formulated with oil.

Furthermore, the present invention provides a transparent solid cosmetic material containing a solid composition using the transparent oil gel as a base material.

### BRIEF DESCRIPTION OF THE DRAWINGS

The accompanying drawings are intended to explain the contents of the present invention in more details to those skilled in the art, but the technical principle of the present invention is not limited thereto.
FIG. 1 shows the results of comparing the wavelength-dependent transmittance of dextrin palmitate prepared according to the manufacturing method of the present invention and a commercial product.
FIG. 2 shows appearance photographs of dextrin palmitate prepared according to the manufacturing method of the present invention and a commercial product.
FIG. 3 shows the results of comparing the wavelength-dependent transparency of a cleansing oil product containing dextrin palmitate prepared according to the manufacturing method of the present invention and a commercial product.

### DETAILED DESCRIPTION OF THE EMBODIMENTS

Hereinafter, the present invention will be described in more details.

The present invention provides a method of preparing a dextrin fatty acid ester, comprising: a first step of dispersing dextrin in an organic mixed solvent containing a hexane-based solvent, and then adding a fatty acid halide while maintaining a temperature of 0 to 120°C to carry out a reaction;
a second step of adding water after the completion of the reaction to induce layer separation and separating the supernatant; and
a third step of precipitating and drying crystals from the supernatant, the organic mixed solvent has different polarities, and in the second step, the hexane-based solvent remains in the supernatant upon phase separation by water, thereby separating the dextrin fatty acid ester.

The method for preparing a dextrin fatty acid ester of the present invention uses an organic mixed solvent having different polarities, and more specifically, the method follows a solvent fractionation method in which the organic mixed solvent includes a first solvent which is a non-polar hexane-based solvent and a second solvent which is a polar solvent, and when the two solvents are mixed, phase separation occurs so that the first solvent having a lower density is positioned as an supernatant, and the second solvent having a higher density becomes a lower layer. Particularly, when water is added to induce phase separation, the lower layer is washed away with the water, and the hexane-based solvent remains in the upper layer, allowing for the separation and purification of the dextrin fatty acid ester.

The first solvent, which is a hexane-based solvent, is any one selected from the group consisting of n-hexane, cyclohexane, and methylcyclohexane. In the embodiments of the present invention, methylcyclohexane is described as a preferred example, but is not limited thereto.

In addition, as an example of the second solvent, which is a polar solvent, any one selected from the group consisting of dimethylformamide, dimethylacetamide, dimethylsulfoxide (DMSO), and N-methyl-2-pyrrolidone (NMP) may be used, and in the embodiments of the present invention, an organic mixed solvent including methylcyclohexane and dimethylformamide is most preferably described as an example, but is not limited thereto.

According to the manufacturing method of the present invention, a dextrin fatty acid ester represented by the following Chemical Formula 1 is prepared and synthesized by reacting dextrin with a fatty acid halide.

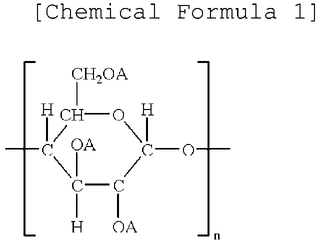

Here, A is a fatty acid, and n is the degree of dextrin polymerization.

In the first step, the dextrin preferably has an average saccharide degree of polymerization of 35 to 80. In this case, when the degree of polymerization of dextrin is less than 35, the dextrin is transparent, but when forming a gel with an oil, the oil separates, and when the degree of polymerization exceeds 80, the dextrin is transparent, but when forming a gel with an oil, the dextrin becomes hard, and the gel shrinks, making it difficult to form a gel suitable for a cosmetic.

The fatty acid is a higher fatty acid having 12 to 22 carbon atoms, preferably one selected from the group consisting of lauric acid (C12), myristic acid (C14), palmitic acid (C16), stearic acid (C18), arachidonic acid (C20), behenic acid (C22), and coconut oil fatty acid (containing 64% of C8 to 12 medium-chain fatty acids), and more preferably, palmitic acid is described as an example in the embodiments of the present invention, but the fatty acid is not limited thereto.

More preferably, according to the manufacturing method of the present invention, dextrin palmitate is prepared, and the gel strength varies depending on the acyl group on the dextrin fatty acid ester, with longer straight-chain fatty acid groups and saccharide chains producing stronger oil gels.

The dextrin palmitate of the present invention has an average degree of acylation substitution of 1.3 to 2.5. In this case, when the degree of substitution is less than 1.3, it is difficult to form an oil gel, and dextrin palmitate tends to not dissolve well, and when the degree of substitution exceeds 2.5, dextrin palmitate dissolves well in an oil and forms a gel, but the gel strength is low.

Accordingly, dextrin palmitate prepared according to the manufacturing method of the present invention satisfies the gel strength requirements depending on the concentration used and the type of oil. Along with its gelling properties for various hydrocarbons and esters, dextrin palmitate provides excellent stability in emulsions.

In the manufacturing method of the present invention, the organic mixed solvent of the first step may further include a catalyst. For example, a picoline-based catalyst such as pyridine or 3-methylpyridine may be used alone or in combination.

After dispersing dextrin in the organic mixed solvent, the fatty acid halide is added dropwise over 15 hours at 10-minute intervals while maintaining a temperature of 0°C to 120°C, and the reaction time is carried out for 1 to 24 hours.

Subsequently, in the second step, after the completion of the reaction in the first step, water is added and stirred for 10 minutes to 6 hours for layer separation. In the third step, a solvent is added to the separated upper layer (oil layer) to precipitate crystals, which are then filtered to produce the target dextrin fatty acid ester.

The solvent used at this time is acetone, methanol, etc. At this time, the polar second solvent in the lower layer (aqueous layer) separated by water is washed away with the water, and the hexane-based solvent remains in the upper layer (oil layer) to separate the dextrin fatty acid ester.

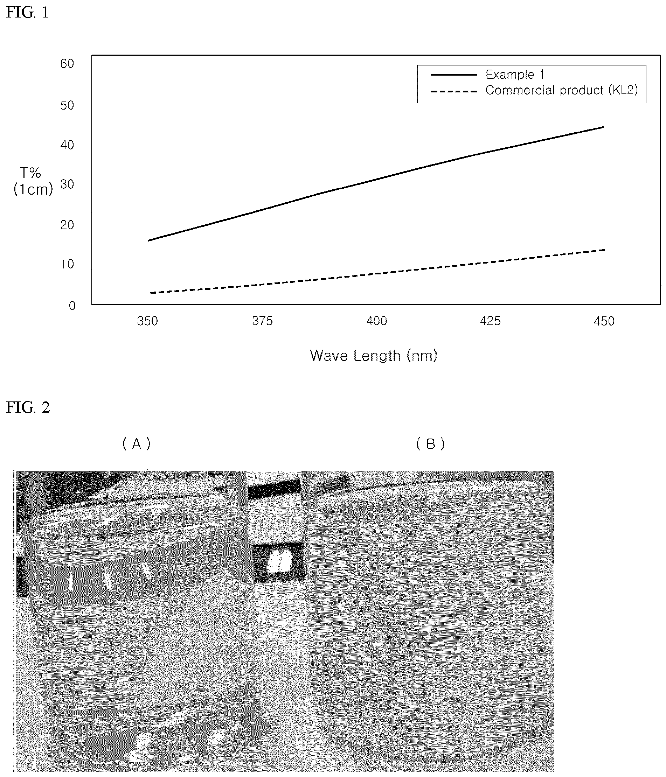

FIG. 1 shows the results of comparing the wavelength-dependent transmittance of dextrin palmitate prepared according to the manufacturing method of the present invention with that of a commercially available dextrin palmitate (KL2, manufactured by Chiba Flour Milling CO., LTD.), confirming the significantly higher transparency of the dextrin palmitate of the present invention.

Specifically, the dextrin palmitate of the present invention has a transmittance of 23.8% at a wavelength of 375 nm (1 cm cell), whereas the commercial product measures 4.8% under the same wavelength condition, confirming that the gel is about 5.0 times more transparent as it transmits 5.0 times more light.

FIG. 2 shows an appearance photograph of the dextrin palmitate of FIG. 1. The transparency of the dextrin palmitate of Example 1 may be visually compared to that of the commercially available dextrin palmitate (KL2, manufactured by Chiba Flour Milling CO., LTD.). Therefore, the dextrin palmitate of the present invention maintains a gel strength of approximately 200 g in a 10% mineral oil gel, while exhibiting transparency more than 5 times that of the comparative example (Leopold KL2, manufactured by Chiba Flour Milling CO., LTD.).

Consequently, according to the method for preparing a dextrin fatty acid ester of the present invention, it is possible to provide a dextrin fatty acid ester, preferably dextrin palmitate, with high gel strength and transparency.

Accordingly, the present invention provides a transparent oil gel including 2% to 20% by weight of a dextrin fatty acid ester prepared using the above-described manufacturing method and 80% to 98% by weight of an oil.

Specifically, the dextrin fatty acid ester is dextrin palmitate, which has a transmittance of 10% or more at a wavelength of 375 nm when formulated with oil. More preferably, in the example, a gel formulated with 10% (w/w) mineral oil is characterized by having a transmittance of 23.8%.

Based on an embodiment of the present invention, the transparent oil gel is prepared by mixing 10% by weight of dextrin palmitate and 90% by weight of mineral oil (Lily 70) as the oil phase, heating to 90°C to dissolve, and then leaving it to stand for 24 hours. At this time, a stable oil gel can be easily obtained by dissolving dextrin palmitate at a high temperature and can be prepared by cooling without stirring. After standing for 24 hours, the gel strength and transparency are measured.

In addition to the above-described oils, significantly high transparency was also confirmed in ethylhexyl palmitate and natural oils such as sunflower oil.

The oil used in the present invention is not particularly limited but triethylhexanoin, hexyl laurate, diethylhexyl succinate, isotridecyl isononanoate, neopentyl glycol diethylhexanoate, isobutyl isostearate, cetyl ethylhexanoate, ethylhexyl palmitate, isostearyl neopentanoate, neopentyl glycol dicaprylate, neopentyl glycol diisononanoate, tri(caprylic/capric) glyceryl, triethylhexanoin, tricyclodecane methyl isononanoate, trimethylol propane triethylhexanoate, octyldodecanol, ethylhexyl hydroxystearate, isostearic acid, octyldodecyl stearoyl oxystearate, diisopropyl dilinoleate, triisostearin, trimethylolpropane triisostearate, pentaerythritol tetraisostearate, polyglyceryl-2 triisostearate, polyglyceryl-2 diisostearate, dipentaerythrityl hexaisononanoate, diisostearyl malate, diisobutyl adipate, hexyldecyl ethylhexanoate, ethylhexyl isononanoate, isodecyl isononanoate, isotridecyl isononanoate, isopropyl myristate, isopropyl palmitate, ethylhexyl palmitate, dipentaerythrityl Hydroxystearate/Isostearate/Rosinate, hydrogenated castor oil isostearate, hydrogenated castor oil dimer dilinoleate, (polyglyceryl-2 isostearate/dimer dilinoleate) copolymers, (polyglyceryl-2 diisostearate/dimer dilinoleate) copolymers, Polyglyceryl-2 Diisostearate/Dimer Dilinoleate Copolymer, Dimer dilinoleate (Phytosteryl/Isostearyl/Cetyl/Stearyl/Behenyl), Bis(Phytosteryl/Behenyl/Isostearyl) Dimer Dilinoleyl Dimer Dilinoleate, Di(Isostearyl/Phytosteryl) Dimer Dilinoleate, Dimer Dilinoleyl Hydrogenated Rosin Condensate, Dimer Dilinoleyl Diisostearate, Dimer Dilinoleyl Dimer Dilinoleate, Di(Cholesteryl/Behenyl/Octyldodecyl) Lauroyl Glutamate, Di(Octyldodecyl/Phytosteryl/Behenyl) Lauroyl Glutamate, (Phytosteryl/Decyltetradecyl) Myristoyl Methylalanine, Diglycerin/Dilinoleic Acid/Hydroxystearic Acid Copolymer, and the like may be used.

Furthermore, the present invention provides a transparent solid cosmetic material containing a solid composition using the transparent oil gel as a base material.

The transparent solid cosmetic material using dextrin palmitate is applicable to various transparent cosmetics, such as transparent lipsticks, transparent lip glosses, transparent lip creams, transparent eye shadows, transparent hair sticks, transparent gel-type perfumes, and transparent makeup sticks.

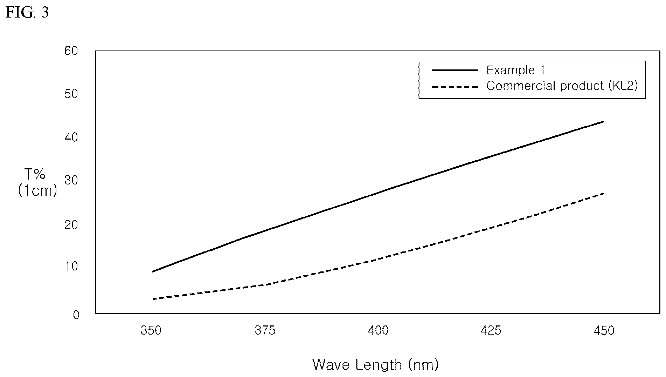

FIG. 3 shows the results of comparing the transparency by wavelength of a product applying the dextrin palmitate prepared according to the manufacturing method of the present invention and a commercial product to cleansing oil. In the case of the cleansing oil containing the dextrin palmitate prepared according to the manufacturing method of the present invention, it was confirmed to be 2.8 times more transparent at a wavelength of 375 nm (1 cm cell) compared to the commercial product.

Hereinafter, the present invention will be described in more detail through examples. These examples are intended to illustrate the present invention in more detail, and the scope of the present invention is not limited to these examples.

### <Example 1> Preparation of dextrin palmitate 1

165 g of dextrin with an average saccharide degree of polymerization of 40 was added to a mixed solvent including 390 g of pyridine, 550 g of dimethylformamide, and 500 g of methylcyclohexane, and then, 570 g of palmitoyl chloride was added to the resulting mixture dropwise over 30 minutes. After completion of the addition, the reaction solution was heated to a reaction temperature of 90°C and stirred for ten hours to complete the reaction. 1,000 ml of purified water was added to the reaction solution, stirred for 30 minutes, and layers were separated. The lower layer was separated and discarded. 50 g of MgSO₄ was added to the upper layer, stirred, and filtered to remove moisture. Acetone was added to the separated upper layer solution to precipitate crystals. The precipitated crystal precipitate was filtered. This was washed with acetone and dried to obtain 600 g of white powder. At this time, the obtained dextrin palmitate had a gel strength of 232 g in a 10% (w/w) mineral oil.

### <Example 2> Preparation of dextrin palmitate 2

The procedure was the same as in Example 1, except that dextrin with an average saccharide degree of polymerization of 60 was used.

### <Example 3> Preparation of dextrin palmitate 3

The procedure was the same as in Example 1, except that dimethyl sulfoxide was used instead of dimethylformamide.

### <Comparative Example 1> Preparation of dextrin palmitate

165 g of dextrin with an average saccharide degree of polymerization of 40 was added to a mixed solvent including 390 g of pyridine and 550 g of dimethylformamide, and 570 g of palmitoyl chloride was then added to the resulting mixture dropwise over 30 minutes. After completion of the addition, the reaction solution was heated to 90°C and stirred for ten hours to complete the reaction. The reaction solution was filtered, and methanol was added to precipitate crystals. The precipitated crystal precipitate was filtered. This precipitate was washed with methanol and dried to obtain 610 g of white powder. The gel strength was 225 g in a 10% (w/w) mineral oil.

### <Experimental Example 1> Transparency evaluation 1

The transparency of the dextrin palmitate obtained in Example 1 and Comparative Example 1 was measured using a transmittance measurement method.

At this time, for this transmittance measurement method, 5 g of the dextrin palmitate obtained in Example 1 and Comparative Example 1 was dissolved in 45 g of mineral oil (Lily 70, Kukdong Oil & Chemical CO., LTD.) at 90°C. The solution was put into a 1 cm cell, placed in an incubator, and maintained at 25°C for 24 hours to perform gelation. The transmittance was then measured at 350 to 500 nm using a spectrophotometer.

### The results are shown in Table 1 below.

**[Table 1]**

| Transparency evaluation results | | | |
|---|---|---|---|
| Classification | Transmittance (%) | | Transmittance ratio |
| Measurement wavelength | Example 1 | Example 2 | |
| 375 nm | 23.8 | 3.5 | 6.8 |
| 400 nm | 31.3 | 5.8 | 5.4 |
| 425 nm | 38.2 | 8.2 | 4.7 |

As shown in Table 1, when a gel was made by adding 10% (w/w) of the dextrin palmitate prepared in Example 1 to mineral oil, the transmittance at a wavelength of 375 nm (1 cm cell) was 23.8%, whereas the one prepared in Comparative Example 1 measured 3.5%. This confirms the gel's transparency, being about 6.8 times more transmissive.

### <Experimental Example 2> Transparency evaluation 2

To compare the transparency of the dextrin palmitate obtained in Example 1, the transmittance of dextrin palmitate of the most widely available commercial product (KL2, manufactured by Chiba Flour Milling CO., LTD.) was measured using the same method as in Experimental Example 1.

The results are shown in Table 2 below, and the results of the comparison are presented in FIGS. 1 and 2.

**[Table 2]**

| Transparency evaluation relative to commercial product | | | |
|---|---|---|---|
| Classification | Transmittance (%) | | Transmittance ratio |
| Measurement wavelength | Example 1 | Commercial product (KL2) | |
| 375 nm | 23.8 | 4.8 | 5.0 |
| 400 nm | 31.3 | 7.5 | 4.2 |
| 425 nm | 38.2 | 10.4 | 3.7 |

As shown in Table 2 above, when a gel was prepared by adding 10% (w/w) of dextrin palmitate prepared in Example 1 to a mineral oil, the transmittance was 23.8% at a wavelength of 375 nm (1 cm cell), whereas the transmittance of the commercial product (KL2, manufactured by Chiba Flour Milling CO., LTD.) was measured to be 4.8% under the same wavelength conditions, confirming that the gel is 5.0 times more transparent as it transmitted about 5.0 times more light.

FIG. 1 shows the transmittance by wavelength for the samples, where the transparency of the dextrin palmitate prepared according to the method of the present invention was remarkable, and FIG. 2 provides a visual comparison of the two samples.

### <Experimental Example 3> Transparency evaluation of product formulations

10 g each of the dextrin palmitate prepared in Example 1 and a commercial product (Chiba Flour Milling Co., Ltd. KL2) were dissolved in 90 g of ethylhexyl palmitate, which is widely used as a cleansing oil, at 90°C. The solution was then placed in a 1 cm cell, kept in an incubator at 25°C for 24 hours to gel, and the transmittance was measured from 350 to 500 nm using a spectrophotometer.

The results are shown in Table 3, and the transmittance results are presented for comparison in FIG. 3.

**[Table 3]**

| Transparency evaluation of product formulations | | | |
|---|---|---|---|
| | Transmittance (%) | | Transmittance ratio |
| Measurement wavelength | Example 1 | KL2 | |
| 375 nm | 16.37 | 5.81 | 2.8 |
| 400 nm | 27.38 | 12.13 | 2.3 |
| 425 nm | 34.25 | 17.24 | 2.0 |

As confirmed in Table 3, the cleansing oil containing the dextrin palmitate prepared in Example 1 was found to be 2.8 times more transparent at a wavelength of 375 nm (1 cm cell) compared to the commercial product.

While the present invention has been described in detail with respect to specific embodiments, it will be apparent to those skilled in the art that various modifications and changes are possible within the scope of the present invention. It is understood that such modifications and changes fall within the scope of the appended claims.

## Claims

1. A method of preparing a dextrin fatty acid ester, comprising:
a first step of dispersing dextrin in an organic mixed solvent containing a hexane-based solvent, and then adding a fatty acid halide while maintaining a temperature of 0 to 120°C to carry out a reaction;
a second step of adding water after the completion of the reaction to induce layer separation and separating the supernatant; and
a third step of precipitating and drying crystals from the supernatant,
wherein the organic mixed solvent has different polarities, and in the second step, the hexane-based solvent remains in the supernatant upon phase separation by water, thereby separating the dextrin fatty acid ester.

2. The method of preparing a dextrin fatty acid ester according to claim 1, wherein the organic mixed solvent includes a first solvent selected from the group consisting of n-hexane, cyclohexane, and methylcyclohexane and a second solvent selected from the group consisting of dimethylformamide, dimethylacetamide, dimethyl sulfoxide (DMSO), and N-methyl-2-pyrrolidone (NMP).

3. The method of preparing a dextrin fatty acid ester according to claim 1, wherein the organic mixed solvent includes methylcyclohexane and dimethylformamide.

4. The method of preparing a dextrin fatty acid ester according to claim 1, wherein the dextrin in the first step has an average saccharide degree of polymerization of 35 to 80.

5. The method of preparing a dextrin fatty acid ester according to claim 1, wherein the fatty acid is a higher fatty acid having 12 to 22 carbon atoms.

6. The method of preparing a dextrin fatty acid ester according to claim 1, wherein the dextrin fatty acid ester is dextrin palmitate.

7. The method of preparing a dextrin fatty acid ester according to claim 6, wherein the dextrin palmitate has an average degree of acylation substitution of 1.3 to 2.5.

8. A transparent oil gel comprising 2 to 20% by weight of a dextrin fatty acid ester prepared by the method of any one of claims 1 to 7, and 80 to 98% by weight of oil.

9. The transparent oil gel according to claim 8, wherein the dextrin fatty acid ester is dextrin palmitate, and the gel has a transmittance of 10% or more at a wavelength of 375 nm when formulated with oil.

10. A transparent solid cosmetic material containing a solid composition using the transparent oil gel of claim 8 as a base material.
